# FASCICULE DE BREVET EUROPEEN

(11) **EP 0 429 823 B1**
(45) Date de publication et mention de la délivrance du brevet: **22.12.1993**
(21) Numéro de dépôt: 90119732.7
(22) Date de dépôt: 15.10.1990
(51) Int. Cl.: A61F 13/08

(54) **Vêtement médical comprenant au moins un bas**
Medizinische Kleidung mit wenigstens einem Strumpf
Medical garment comprising at least one stocking

(30) Priorité: 01.12.1989 CH 4291/89
(43) Date de publication de la demande: 05.06.1991
(73) Titulaire: Parvulesco, Jean, CH-1844 Villeneuve (CH)
(72) Inventeur: Parvulesco, Jean, CH-1844 Villeneuve (CH)
(74) Mandataire: Misrachi, Alfred

(56) Documents cités:
- DE-A- 2 007 860
- US-A- 4 424 596

## Description

La présente invention a pour objet un vêtement médical comprenant au moins un bas élastique ouvert à ses extrémités destiné à créer une contention sur tout ou partie d'un membre inférieur.

Les bas élastiques médicaux, couramment utilisés en thérapeutique, voir pour exemple US-A-4 424 596, en classes II à IV, par exemple pour le traitement d'oedèmes de toutes sortes ou de grosses jambes de natures diverses, et qui bénéficient du remboursement par les caisses maladie, ont tous le bout du pied ouvert.

Le rôle de ces bas est d'exercer une pression sur la surface cutanée du membre de manière dégressive à partir de son extrémité inférieure, l'effet de compression maximale se situant ainsi autour de la moitié antérieure du pied.

Malheureusement dans la pratique ces bas sont confrontés à un problème majeur de glissement postérieur par le fait que lors des mouvements du patient leur extrémité inférieure tend à se déplacer vers le talon, en laissant ainsi à découvert la moitié antérieure du pied, c'est-à-dire justement la région devant subir la compression la plus forte, ce qui va à rebours du sens de la compression dégressive recherchée. En outre, par son glissement, le bas peut faire des plis dans la région tarsienne, en créant parfois un vrai phénomène de garrot qui bloque la circulation de retour et provoque une augmentation de l'oedème et des douleurs à ce niveau.

L'invention a pour but un vêtement médical grâce auquel les inconvénients précités des bas connus sont éliminés.

Dans la préoccupation d'améliorer ainsi la qualité de la contention chez les malades phlébopathiques, ce vêtement médical, du genre décrit en début d'exposé, est caractérisé en ce que le bas comporte à son extrémité ouverte correspondante aux orteils une partie fermée destinée à recevoir le gros orteil.

De la sorte, retenu par cette partie fermée, le bas du vêtement est fixé au niveau de la racine des orteils et il est empêché de se déplacer pendant les mouvements du patient.

Susceptible d'être utilisé notamment en phlébologie et en chirurgie, ce vêtement présente l'avantage, par rapport à ceux connus du même genre, d'une stabilité accrue, d'une compression bien dosée à partir de la racine des orteils et d'un port facile et agréable pour le patient par la suppression des plis qu'il permet, ce qui répond bien au but de l'invention.

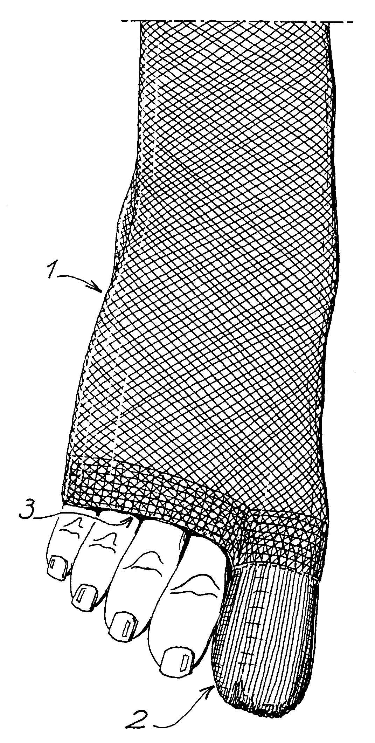

Le dessin annexé représente, à titre d'exemple, une forme d'exécution de l'objet de l'invention.

L'unique figure en est une représentation partielle.

La partie du vêtement représentée est la partie inférieure d'un bas élastique de contention 1, recouvrant un membre inférieur d'un patient.

Le bas 1 comporte à son extrémité inférieure une partie fermée 2 en forme de doigtier recouvrant le gros orteil et une partie ouverte 3 au niveau de la racine des autres orteils, laissant ceux-ci à découvert.

Ce bas 1 est confectionné comme tout bas médical et suivant un procédé approprié à créer une contention, par exemple en gomme élastique synthétique du genre élasthane.

La partie fermée 2 en forme de doigtier est ici confectionnée par un tricot contenant des fils élastiques disposés longitudinalement, afin de constituer une retenue sans effet de compression sur le gros orteil.

Le vêtement médical comprenant au moins un bas de ce genre peut se présenter selon plusieurs variantes, non représentées par le dessin. Il peut ainsi recouvrir pied et jambe - pied, jambe et mi-cuisse - pied, jambe et cuisse - pied, jambe, cuisse et ceinture abdominale; il peut aussi comporter deux bas et constituer un collant.

Par ses qualités de compression bien répartie à partir de la racine des orteils, de stabilité accrue et de facilité de port, le vêtement ainsi conçu peut fournir un soutien médical approprié et il a des indications thérapeutiques multiples telles que :
a) Les oedèmes des membres inférieurs dont la guérison ne peut plus être obtenue par un traitement actif, comme par exemple les oedèmes survenant lors d'insuffisance veineuse superficielle, lors d'insuffisance veineuse profonde, notamment les oedèmes post-thrombotiques, et les oedèmes lymphatiques.
b) Après la chirurgie des varices.
c) Le post-traitement sclérosant des varices, notamment au niveau du pied.
d) Les grosses jambes de natures diverses.

## Revendications

1. Vêtement médical comprenant au moins un bas élastique (1) ouvert à ses extrémités destiné à créer une contention sur tout ou partie d'une membre inférieur, caractérisé en ce que le bas (1) comporte à son extrémité ouverte (3) correspondante aux orteils une partie fermée destinée à recevoir le gros orteil.

2. Vêtement selon la revendication 1, caractérisé en ce que la partie (2) destinée à recevoir le gros orteil est un tricot contenant des fils élastiques disposés longitudinalement.

3. Vêtement selon l'une des revendications 1 et 2, caractérisé en ce qu'il s'étend jusqu'à la mi-cuisse ou la cuisse.

4. Vêtement selon l'une des revendications 1 et 2, caractérisé en ce qu'il s'étend jusqu'à la ceinture abdominale.

5. Vêtement selon l'une des revendications 1 et 2, caractérisé en ce qu'il comprend deux bas et constitue un collant.

## Claims

1. Medical clothing comprising at least one elastic stocking (1) open at its ends intended to create a containment on all or part of a lower member, characterized in that the stocking (1) comprises at its open end (3) corresponding to the toes a closed part intended to receive the big toe.

2. Clothing according to claim 1, characterized in that the part (2) intended to receive the big toe is a knitting containing longitudinally arranged elastic threads.

3. Clothing according to any of claims 1 and 2, characterized in that it extends up to the mid-thigh or the thigh.

4. Clothing according to any of claims 1 and 2, characterized in that it extends up to the abdominal girdle.

5. Clothing according to any of claims 1 and 2, characterized in that it comprises two stockings and constitute tights.

## Patentansprüche

1. Medizinisches Kleidungsstück mit wenigstens einem an seinen Enden offenen elastischen Strumpf (1) zum Bilden einer stützenden Umfassung für die Gesamtheit oder einen Teil eines unteren Körpergliedes, dadurch gekennzeichnet, daß der Strumpf (1) an seinem offenen, die Zehen aufnehmenden Ende (3) einen geschlossenen Abschnitt für die Aufnahme des großen Zehs aufweist.

2. Kleidungsstück nach Anspruch 1, dadurch gekennzeichnet, daß der der Aufnahme des großen Zehs dienende Abschnitt (2) aus einer Maschen- oder Webware mit in Längsrichtung angeordneten elastischen Fäden gebildet ist.

3. Kleidungsstück nach einem der Ansprüche 1 und 2, dadurch gekennzeichnet, daß es sich über die Mitte des Oberschenkels oder über den Oberschenkel erstreckt.

4. Kleidungsstück nach einem der Ansprüche 1 und 2, dadurch gekennzeichnet, daß es sich bis zum Bauchbereich erstreckt.

5. Kleidungsstück nach einem der Ansprüche 1 und 2, dadurch gekennzeichnet, daß es zwei Strümpfe umfaßt und eine Strumpfhose bildet.
